# EUROPEAN PATENT APPLICATION

(11) **EP 1 001 016 A1**
(43) Date of publication of application: **17.05.2000**
(21) Application number: 99911820.1
(22) Date of filing: 06.04.1999
(51) Int. Cl.: C12N 1/20, A61K 39/106, C12N 1/36

(54) **ANTI-$i(VIBRIO ANGUILLARUM) VACCINE (GAVA-3) FOR THE PREVENTION OF THE VIBRIOSIS DISEASE IN THE TURBOT AND SALMONIDAE, AND PREPARATION PROCESS**

(30) Priority: 27.04.1998 ES 9800889
(71) Applicant: UNIVERSIDADE DE SANTIAGO DE COMPOSTELA, 15705 Santiago de Compostela (ES)
(72) Inventor: SANTOS RODRIGUEZ, Ysabel, Uni. Santiago Compostela, 15706 Santiago de Compostela (ES); ESTEVEZ TORANZO, Alicia, Uni. Santiago Compostela, E-15706 Santiago de Compostela (ES); BARJA PEREZ, Juan Luis, Uni. Santiago Compostela, E-15706 Santiago de Compostela (ES)
(74) Representative: Davila Baz, Angel
(86) International application number: ES9900089
(87) International publication number: WO9955835

(57) **Abstract**

The invention relates to an anti-*Vibrio anguillarum* vaccine (GAVA-3) for the prevention of vibriosis in the turbot and Salmonidae fish and the process for producing such vaccine. The vaccine is comprised of three bacterial strains of *Vibrio angillarum* isolated from a turbot: strain CECT 5031 (serotype 01), strain CECT 5032 (serotype 02, sub-group 02α) and CECT 5033 (serotype 02, sub-group 02β), and is also comprised of their extracellular products. Both the strains and the extracellular products are inactivated with formol. The vaccine applies to the field of aquaculture.

## Description

Anti-*Vibrio anguillarum* vaccine (GAVA-3) for the prevention of the vibriosis disease in turbot and salmonid fishes, and preparation process.

The objective of this patent of invention is to present a new vaccine to protect turbot and salmonid fish cultured in seawater against vibriosis based on the mixture of cell, and extracellular products from pathogenic *V. anguillarum* strains of the serotypes O1 and O2 (subgroups O2α and O2β). The advantage of this vaccinewith regard to other *Vibrio* vaccines is that it covers a higher antigenic spectrum, giving to the fishes a high degree of proteection (80%) against the serotype O1 and the two antigenic subgroups of the serotype O2 (O2α and O2β).

The vibriosis, caused mainly by *V. anguillarum* species, is one of the most important bacterial diseases that affect marine fishes, molluscs and crustacea over the world. Since its first description in turbot cultured in Galicia (Northwest of Spain) in 1985 (Devesa et al, 1985: In "Fish and Shellfish Pathology, A E. Ellis, ed. Chapter XIV pp131-140, Academic Press) this disease has become in one of the main pathological problems of the marineculture of our area. Although a total of ten serotypes have been described in this bacterial species, only serotypes O1 and O2 have been associated with fish mortalities. (Toranzo et al, (1987): Aquaculture,67:41-51; Santos et al (1995): Applied and Environmental Microbiology, 61:2495-2498). Moreover, while the serotype O1 constitutes a phenotypic and genetic homogeneous group, the serotype O2 shows heterogeneity at biochemical, serological and genetic level, with two antigenic entities been described (O2α andO2β) (Toranzo et al, (1987); Santos et al (1995) opus cited). Moreover, it has been demonstrated that the extracellular products from strains of serotype O1 and the two antigenic subgroups of serotype O2 (O2α andO2β) are highly toxic for fish, show qualitative and/or quantitative differences in their enzymatic composition and are immunologically differents (Santos at al (1991): Journal of Applied Ichthyology, 7:160-167; Santos at al., (1995) op. cited. This information has been considered to select the vaccine components. The strains of *V. anguillarum* included in this vaccine are the isolates R-82 (serotype O1), RG-111 (scrotype O2, subgroup O2α) and RV-22 (serotype O2, subgroup O2β) and have been deposited by the inventors of this patent in the Spanish Type Culture Collection with the accession numbers CECT5031, CECT5032 and CECT 5033, respectively. These strains present the typical characteristics of the species, being halophilic rods Gram-negatives, motiles, oxidase positives, fermentatives and senitives to the vibriostatic agent O/129.

With regard to the extracellular products, the samples from the three strains show hemolytic, proteolytic and cytotoxic activities. The strains CECT 5031 y CECT 5032 also display valine arylamidase, trypsin, chemotrypsin and dermonecrotic activities which are absent in the strain CECT 5033. The extracellular products of the three strains possess proteic and lipopolysaccharide components immunologically distinct (Santos at al (1995) op. cited.

### PREPARATION

Bacterial cultures on logaritmic phase of each strain are used to inoculate trypticase soy broth medium with 1% of NaCl added (TSC-1, tryptone 17 gr/L; soya peptone 3 gr/L; glucose 2,5 gr/L; di-potassium hydrogen phosphate 2,5 gr/L; NaCl 10 gr/L; pH 7,3± 0,2).Cultures are incubated at 25°C for 48h. After the incubation period, formalin (0,35%) are added to the cultures to kill the bacteria and to inactivate the extracellular products. The inactivated cultures are mantained during three hours more at 25°C with shaking and after that they are refrigerated at 4°C. Inactivated cultures of the three strains used in the vaccine are adjusted to an optical density of 1 (A₅₅₀, Absorbance ₅₅₀) and mixed in a 50:25:25% proportion (CECT 5031:CECT 5032:CECT 5033). The mixed cultures contained aproximatelly 10¹⁰ cells/ml and 160µg of extracellular protein/ml.

The control of specificity is performed by slide agglutination using antisera raised in rabbit against each of the strain used for vaccine production (CECT 5031 :CECT 5032:CECT 5033) and whole cells suspensions of cited strains as antigen.

The product should be kept at 4°C until use.

### ADMINISTRATION SCHEDULES

For deep administration, the vaccine is diluted 1/10 in water and the fish are introduced in this vaccine bet during 1 min with aireation. With each liter it can be vaccinated 100Kg/fish in 20 series of 5 Kg each. Fish can be also vaccinated by immersion during 1 h with aireation in a vaccine dilution of 1/1000. To administrate the vaccine by injection, fish are inoculated with 0.1-0.2 ml/fish (depending of the fish weight) of undiluted vaccine.

To improve the efficacy of the vaccine it is recommendable to immunize fish of 1 g of body weight and perform a booster vaccination 4 weeks later.

The strains of *Vibrio anguillarum* used in this vaccine are R-82 (serotype O1), RG-111 (scrotype O2, subgroup O2α and RV-22 (serotype O2, subgroup O2β) and have been isolated from turbot cultured in Galicia (Northwest of Spain).

The efficacy of this vaccine has been evaluated at laboratory and industrial level. The potency, expressed as relative percentage of survival (RPS) is higher than 80%.

## Claims

1. Strains of *Vibrio anguillarum* R-82 (serotype O1), RG-111 (serotype O2, subgroup O2α) and RV-22 (serotype O2, subgroup O2β) isolated from turbot cultured in Galicia (Northwest of Spain), deposited in the Spanish Type Culture Collection with the accession numbers CECT5031, CECT5032 and CECT 5033, respectively.

2. Vaccine anti-*Vibrio anguillarum* to protect turbot and salmonid fish cultured in seawater against the vibriosis caused by the serotypes O1 and O2 (antigenic subgroups O2α and O2β) composed by the bacterial cells and the extracellular products inactivated by formalin of the strains CECT5031 (serotype O1), CECT5032, (serotype O2, subgroup O2α) and CECT 5033 (serotype O2, subgroup O2β) of this microorganism.

3. Procedure to prepare the vaccine cited on the claim 2, characterized by the culture of the strains in the liquid medium Trypticase Soy broth suplemented with 1% of NaCl, by the inactivation of the bacterial cells and their extracellular products by treating with formalin (0,35%) during three hours at 25°C and because the inactivated cultures of the strains are adjusted to an optical density of 1 (A₅₅₀ Absorbance ₅₅₀).

4. Procedure following the claims 2 and 3 and characterized by the fact that the final proportion of the mixture of the strains and their extracellular products in the vaccine is %50:25: 25% (CECT 5031:CECT 5032:CECT 5033).

5. Procedure following the claims 2 and 3 and 4, characterized by the fact that the bacterial concentration in the vaccine preparation is 10¹⁰ cells/ml and the protein concentration of extracellular products is 160µg/ml.

6. Procedure for the administration of the vaccine of the claim 2, characterized by the fact that the vaccine can be administrated by dip (1 min in vaccine diluted 1/10), bath (1 hour in vaccine diluted 1/1000), or by intraperitoneal injection of undiluted vaccine using doses of 0.1 ml, for fish of 20 grs of body weight, or 0.2 ml for higher fishes.

## Amended claims

### Amended claims under Art. 19.1 PCT

1. Strains of *Vibrio anguillarum* R-82 (serotype O1), RG-111 (serotype O2, subgroup O2α) and RV-22 (serotype O2, subgroup O2β) isolated from turbot cultured in (Galicia (Northwest of Spain), deposited in the Spanish Type Culture Collection with the accession numbers CECT5031, CECT5032 and CECT 5033, respectively.
2. Vaccine anti-*Vibrio anguillarum* to protect turbot and salmonid fish cultured in seawater against the vibriosis caused by the serotypes O1 and O2 (antigenic subgroups O2α and O2β) composed by the bacterial, cells and the extracellular products inactivated by formalin of the strains CECT5031 (serotype O1), CECT5032 (serotype O2, subgroup O2α) and CECT 5033 (serotype O2, subgroup O2β) of this microorganism.
3. Procedure to prepare the vaccine cited on the claim 2, characterized by the culture of the strains in the liquid medium Trypticase Soy broth suplemented with 1% of NaCl, by the inactivation of the bacterial cells and their extracellular products by treating with formalin (0,35%) during three hours at 25°C and because the inactivated cultures of the strains are adjusted to an optical density of 1 (A₅₅₀, Absorbance ₅₅₀).
4. Procedure to prepare the vaccine anti-*Vibrio anguillarum* cited on claims 2 and 3, characterized by the fact that the final proportion of the mixture of the strains and their extracellular products in the vaccine is %0:25: 25% (CECT 5031:CECT 5032:CECT 5033).
5. Procedure to prepare the vaccine anti-*Vibrio anguillarum* cited on claims 2, 3 and 4, characterized by the fact that the bacterial concentration in the vaccine preparation is 10¹⁰ cells/ml and the protein concentration of extracellular products is 160µg/ml.
